# EUROPEAN PATENT APPLICATION

(11) **EP 4 461 307 A1**
(43) Date of publication of application: **13.11.2024**
(21) Application number: 22918327.2
(22) Date of filing: 05.12.2022
(51) Int. Cl.: A61K 38/48, C07K 14/33, A61K 9/08, A61K 47/26, A61K 47/18, A61P 25/06, A61P 19/08, A61P 27/02, A61P 17/00, A61P 21/02, A61P 25/00, A61P 29/02, A61Q 19/08

(54) **BOTULINUM TOXIN PROTEIN COMPOSITION**

(30) Priority: 07.01.2022 CN 202210018289
(71) Applicant: Chongqing Claruvis Pharmaceutical Co., Ltd., Chongqing 400713 (CN)
(72) Inventor: YANG, Wu, Chongqing 400713 (CN); GONG, Hui, Chongqing 400713 (CN)
(74) Representative: HGF
(86) International application number: PCT/CN2022/136597
(87) International publication number: WO 2023/130875

(57) **Abstract**

A botulinum toxin protein composition, comprising a botulinum toxin protein and a protein stabilizer. The protein stabilizer is selected from any one of amino acids or amino acid-like compounds, the amino acids being one or more of L-histidine, L-glycine, L-arginine, L-methionine and L-lysine, and the amino acid-like compounds comprising L-carnitine. The botulinum toxin protein composition can be used as a liquid preparation in the fields of medical treatment and beauty. The activity of the botulinum toxin protein in a liquid state can be kept stable.

## Description

### TECHNICAL FIELD

The present invention relates to the field of pharmaceutical formulations, and in particular, to a botulinum toxin protein composition and a method for preparing same.

### BACKGROUND

Native botulinum neurotoxins (BoNTs) are toxin proteins produced by an anaerobic bacterium *Clostridium botulinum.* They cause paralysis in the muscles of mammals by blocking the presynaptic release of neurotransmitter acetylcholine at the neuromuscular junction. Seven BoNTs have been identified at present, named BoNT/A to BoNT/G, respectively. BoNTs have rapidly become therapies for cholinergic hyperexcitability due to their unique pharmacological properties including high specificity for the nervous system, limited diffusion by local injection, and reversible effects, and possess broad applications in the fields of medicine and cosmetology.

In general, therapeutically and pharmaceutically active proteins are prepared in solutions, in particular for injections. Therapeutically active proteins are generally prepared as compositions and commercialized as a ready-to-use solution or a lyophilized form for reconstitution into a solution. For example, commercial BoNT/A products are vacuum-dried or lyophilized solid formulations and require reconstitution by healthcare providers at the time of use, thus requiring precise control of the reconstitution volume. In addition, due to the differences in therapeutic purpose and individual dosage between patients, the volume required for each dose varies considerably. After reconstituting the commercial products to the volume specified by the manufacturer, clinicians often administer only a small portion of the content in the vial to the patient, and preserve the remainder in a refrigerator for later use. It has been estimated that the preservation of a reconstituted BoNT/A formulation in a refrigerator (about 4 °C) for 12 h may lead to a 44% or greater loss of efficacy. Thus in practice, it is generally recommended to use the product within 4 h after reconstitution, which results in a waste of medications and an unnecessary economic burden for the patient. In addition, due to the extremely low concentration of botulinum toxin in use, it is generally prepared with the addition of high concentrations of exogenous proteins to keep the stability of the botulinum toxin protein at the low concentration and avoid the adsorption of the active ingredient onto solid surfaces. In most botulinum toxin formulations, albumin or gelatin is used as the stabilizer, which not only causes immune responses, but also has the potential to introduce a source of contamination.

### SUMMARY

In view of the technical problems put forward in the BACKGROUND, the present invention provides a botulinum toxin protein composition and a method for preparing same.

The embodiments of the present invention are as follows:
The present invention provides a botulinum toxin protein composition, comprising:
a botulinum toxin protein and a protein stabilizer.

The protein stabilizer is selected from any one of an amino acid or an amino acid analog;
preferably, the amino acid analog is L-carnitine (also known as vitamin Bt).

More preferably, the molar content of L-carnitine is 5-20 mM, and in one specific embodiment, the molar content of L-carnitine is 10 mM.

Preferably, the molar content of the amino acid is 5-20 mM; and in one specific embodiment, the molar content of the amino acid is 10 mM.

More preferably, the amino acid is selected from one or more of L-histidine, L-glycine, L-arginine, L-methionine, L-histidine, and L-lysine.

In general, proteins are highly susceptible to degradation and inactivation at low concentrations (<0.1 mg/mL). Some amino acids and amino acid analogs in the present application can increase the solubility of proteins at specific pH values and improve their stability. In addition, the amino acids and amino acid-like structural compounds not only reduce the surface adsorption and protect the conformation of proteins, but also prevent the denaturation and aggregation of proteins. Moreover, when the protein stabilizer in the present invention is L-carnitine, compared with commonly used auxiliary materials, L-carnitine gives the formulation the characteristic of low viscosity and reduces the aggregation and sedimentation of the botulinum toxin protein, which is beneficial to maintaining the stability of botulinum toxin protein.

Preferably, the botulinum toxin protein is selected from any one of botulinum toxin type A protein, botulinum toxin type B protein, botulinum toxin type C1 protein, botulinum toxin type C2 protein, botulinum toxin type D protein, botulinum toxin type E protein, botulinum toxin type F protein, or botulinum toxin type G protein; more preferably, the botulinum toxin protein is botulinum toxin type A protein.

More preferably, the botulinum toxin is a recombinant botulinum toxin.

More preferably, the recombinant botulinum toxin protein has at least one dimeric structure composed of polypeptides formed from a single-chain polypeptide cleaved by a protease;
The single-chain polypeptide comprises:
(I) a first polypeptide fragment comprising:
   (a) a protein tag,
   (b) a structural region comprising a first protease cleavage site; and
   (c) a short linker peptide; and
(II) a second polypeptide fragment comprising:
   (d) a first functional amino acid structural region comprising a metal ion-dependent protease activity domain;
   (c) a structural region comprising a second protease cleavage site; and
   (f) a second functional amino acid structural region comprising a receptor binding domain that can bind to a surface receptor of a target cell and/or a translocation domain that can mediate the transfer of the polypeptide across a vesicle membrane. The term "protein tag" refers to a class of protein molecules that can bind to a specific ligand.

Preferably, the protein tag is selected from a protein tag known to those skilled in the art or a protein tag designed by a computer program, and is capable of specifically binding to a known substrate.

More preferably, theprotein tag is selected from, but not limited to, the following proteins: glutathione S-transferase (GST), C-myc, chitin-binding domain, maltose-binding protein (MBP), SUMO heterologous affinity moiety, monoclonal antibody or protein A, streptavidin-binding protein (SBP), cellulose-binding domain, calmodulin-binding peptide, S tag, Strep tag II, FLA, protein A, protein G, histidine affinity tag (HAT), and polyhistidine.

Preferably, the "protein tag" can increase the solubility of a toxin polypeptide precursor in a host.

Preferably, the "protein tag" allows the presence of a toxin polypeptide precursor in a host cell in a soluble manner.

In one specific embodiment, the protein tag is located at the N terminus of the single-chain polypeptide.

In one specific embodiment, the protein tag is glutathione S-transferase (GST), and more preferably, the amino acid sequence of glutathione S-transferase is set forth in SEQ ID NO: 2.

Preferably, neither the first protease cleavage site nor the second protease cleavage site can be cleaved by a human protease or a protease produced by the host cell expressing the single-chain polypeptide.

More preferably, the first protease cleavage site and the second protease cleavage site are identical or different.

The first protease cleavage site and the second protease cleavage site are not recognized and cleaved by an endogenous protease of a host cell when expressed or an organism when used.

More preferably, the specific protease is selected from, but not limited to, one of or a combination of two of the following proteases: a non-human enterokinase, a tobacco etch virus protease, a protease derived from *Bacillus subtilis,* a protease derived from *Bacillus amyloliquefaciens,* a protease derived from rhinovirus, papain, a homologue of papain of an insect, or a homologue of papain of a crustacean.

In one specific embodiment, the protease that specifically recognizes the first protease cleavage site and the protease that specifically recognizes the second protease cleavage site are both derived from the protease of rhinovirus.

Preferably, the second enzyme cleavage site is embedded into, partially replaces, or completely replaces a natural loop region between a first functional peptide fragment and a second functional peptide fragment. The embedding refers to an embedding between two certain amino acids in the loop region; the partial replacement refers to that the second enzyme cleavage site replaces part of the amino acid sequence of the loop region; the complete replacement refers to that the amino acid sequence of the natural loop region is completely replaced by the second enzyme cleavage site.

More preferably, the structural region comprising the first protease cleavage site and the structural region comprising the second protease cleavage site are selected from, but not limited to, one of or a combination of two of the following enzyme cleavage sites: DDDDK, EXXYXQS/G, HY, YH, or LEVLFQGP.

In a specific embodiment, the structural region comprising the first protease cleavage site and the structural region comprising the second protease cleavage site are both LEVLFQGP, and the cleavage sites are between Q-G.

Preferably, the short linker peptide has no more than 5 amino acids.

Preferably, the short linker peptide enables the first protease cleavage site to be more easily recognized or bound to by a protease thereof.

More preferably, the short linker peptide does not affect the function of the single-chain polypeptide.

More preferably, the short linker peptide retained at the N terminus of the second polypeptide fragment does not affect the function of the second polypeptide fragment after the cleavage of the first protease cleavage site.

More preferably, a GS part of the short linker peptide has no more than 5 amino acid residues, and even more preferably, the short linker peptide is selected from glycine-serine (or GS) short peptide, GGS, GGGS, GGGGS, GSGS, GGSGS, GSGGS, GGSGS, GGGSS, and other short linker peptides.

In one specific embodiment, the amino acid sequence of the structural region comprising the first protease cleavage site and the short linker peptide is LEVLFQGPLGS.

Preferably, the metal ion-dependent protease activity domain is a Zn²⁺-dependent protease activity domain.

Preferably, the first functional amino acid structural region and/or the second functional amino acid structural region are encoded by a natural sequence and/or an artificially synthesized sequence. More preferably, the first functional amino acid structural region of the single-chain polypeptide comprises a Zn²⁺ protease activity domain of the light chain of a clostridial neurotoxin.

Preferably, the target cell of the receptor-binding domain capable of binding to an antigen on the surface of a human cell in the second functional amino acid structural region refers to a target cell with an SNARE complex. For example, a nerve cell, a pancreatic cell, or other cells with an SNARE complex.

More preferably, the target cell of the second functional amino acid structural region refers to a human nerve cell or a pancreatic cell, and the receptor-binding domain is a receptor-binding domain capable of specifically binding to the human nerve cell or the pancreatic cell.

More preferably, the receptor-binding domain of the second functional amino acid structural region is a cell surface antigen-binding domain of the heavy chain of clostridial neurotoxin, and the translocation domain of the second functional amino acid structural region that mediates the transfer of the polypeptide across the vesicle membrane is a domain that mediates the transfer of clostridial neurotoxin across the vesicle membrane.

More preferably, the clostridial neurotoxin is a botulinum neurotoxin or tetanus toxin. More preferably, the botulinum neurotoxin is selected from any one of serotypes BoNT/A-BoNT/H and derivatives thereof known in the art.

More preferably, the clostridial neurotoxin is selected from any one of tetanus toxin or a derivative thereof.

More preferably, the first functional amino acid structural region comprises part or all of the light chain of BoNT/A, BoNT/B, BoNT/C, BoNT/D, BoNT/E, BoNT/F, BoNT/G, BoNT/H or tetanus toxin.

More preferably, the second functional amino acid structural region comprises part or all of the heavy chain of BoNT/A, BoNT/B, BoNT/C, BoNT/D, BoNT/E, BoNT/F, BoNT/G, BoNT/H or tetanus toxin.

In one specific embodiment, the first functional amino acid structural region is the light chain of BoNT-/A, and the second functional amino acid structural region is the heavy chain of BoNT-/A.

More preferably, the first functional amino acid structural region and the second functional amino acid structural region may be from different serotypes. The first functional amino acid structural region and the second functional amino acid structural region may be any combination of the above serotypes. For example, the first functional amino acid structural region is derived from the light chain of BONT-/A, and the second functional amino acid structural region is derived from the heavy chain of BONT-/B, BoNT/C, BoNT/D, BoNT/E, BoNT/F, or BoNT/G, or the first functional amino acid structural region is derived from the light chain of BoNT/B, BoNT/C, BoNT/D, BoNT/E, BoNT/F or BoNT/G and the second functional amino acid structural region is derived from the heavy chain of BoNT/A, and the like.

More preferably, the second polypeptide fragment has an amino acid sequence comprising a fragment set forth in SEQ ID NO: 7.

In one specific embodiment, the single-chain polypeptide sequentially comprises, from the N terminus, glutathione S-transferase, LEVLFQGPLGS, the light chain of BoNT/A, LEVLFQGP, and the heavy chain of BoNT/A.

More preferably, the single-chain polypeptide has an amino acid sequence set forth in SEQ ID NO: 11.

More preferably, the recombinant botulinum toxin type A protein is prepared by cleaving the single-chain polypeptide with a first protease to remove the protein tag, and forming at least one dimer with the first functional amino acid structural region and the second functional amino acid structural region after cleavage with a second protease.

Preferably, the first functional amino acid structural region and the second functional amino acid structural region are connected to form a dimeric structure through a disulfide bond.

The recombinant botulinum toxin type A protein of the present invention is formed by a specific single-chain polypeptide, wherein the related single-chain polypeptide has a specific structure, and the single-chain polypeptide of such a structure has the characteristics of strong specificity and low toxicity. Specifically, from the perspective of strong specificity, the protein tag in the single-chain polypeptide can bind to a specific ligand and subsequently be separated from the ligand, so that the molecule co-expressed with the protein tag can be selected from a variety of polypeptides or protein products more easily; in the preparation process, the purity of the product can be remarkably improved, thus giving a higher product yield and purity. From the perspective of low toxicity, the protein tag in the single-chain polypeptide prevents the protease structural domain of the first functional amino acid structural region in the polypeptide from exerting its protease functionality; by replacing the natural enzyme cleavage site between the first and second functional amino acid structural regions with a site recognized only by a specific enzyme, the molecular activity (neurotoxicity) of the single-chain polypeptide is reduced to one ten-thousandth or less that of the natural toxin, thereby effectively reducing the toxicity; due to the greatly reduced toxicity, the safety of the production process is improved, making the entire industrial production easier to operate. That is, the single-chain polypeptide in the recombinant botulinum toxin protein related to the present invention is easier for industrial production and purification due to the higher yield, purity, and safety as compared with the prior art. Preferably, the botulinum toxin protein composition further comprises a solubilizer and/or a salt compound.

More preferably, the volume percentage concentration of the solubilizer is 0.05%-0.2%.

More preferably, the mass percentage concentration of the salt is 0.9%.

More preferably, the solubilizer is selected from a Tween, a Span, and sodium dodecyl sulfate;
more preferably, the Tween is selected from one or more of Tween 20, Tween 40, Tween 60, or Tween 80;
even more preferably, the Span is selected from one or more of Span 20, Span 40, Span 60, Span 80, or Span 85;
in one specific embodiment, the solubilizer is Tween 80.

Preferably, the salt is selected from any one of an inorganic salt or an organic salt; more preferably, the inorganic salt is selected from one or more of sodium chloride, sodium phosphate, magnesium sulfate, sodium acetate, sodium propionate, and tripotassium phosphate;
even more preferably, the organic salt is selected from one or more of sodium lactate and sodium succinate;
in one specific embodiment, the inorganic salt is sodium chloride.

Preferably, the composition comprises the botulinum toxin protein, L-carnitine, Tween 80, and sodium chloride;
or,
the composition comprises the botulinum toxin protein, the amino acid, Tween 80, and sodium chloride.

More preferably, the molar content of L-carnitine is 5-20 mM, preferably 10 mM;
or, the molar content of the amino acid is 5-20 mM, preferably 10 mM.

More preferably, the volume percentage concentration of Tween 80 is 0.05%-0.2%. In one specific embodiment, the volume percentage concentration of Tween 80 is 0.1%. More preferably, the mass percentage concentration of sodium chloride is 0.9%.

The composition is a liquid preparation.

The present invention further provides a method for preparing a botulinum toxin protein composition, comprising a step of mixing the components.

Preferably, the method described above comprises:
(a) preparing and purifying a botulinum toxin protein; and
(b) adding a protein stabilizer solution to the botulinum toxin protein.

The solution is solution for injection.

More preferably, step (b) further comprises adding a solubilizer and/or a salt.

The botulinum toxin protein, the protein stabilizer, the solubilizer, and/or the salt are as defined above.

In one specific embodiment, the method comprises:
(a) preparing and purifying a recombinant botulinum toxin type A protein; and
(b) adding a protein stabilizer solution, Tween 80, sodium chloride and water into the recombinant botulinum toxin type A protein.

The solution is solution for injection.

The present invention further provides use of the botulinum toxin protein composition described above in preparing a medicament for preventing and/or treating a disease associated with cholinergic nerves innervating muscles or exocrine glands or a medicament for medical cosmetology.

The disease or condition associated with cholinergic nerves innervating muscles or exocrine glands includes exemplary diseases or conditions of muscular or exocrine gland hyperactivity associated with cholinergic innervation, including, e.g., Frey's syndrome, crocodile tears syndrome, armpit hyperhidrosis, plantar hyperhidrosis, head and neck hyperhidrosis, body hyperhidrosis, rhinorrhea, Parkinson's disease, amyotrophic lateral sclerosis, sialorrhea, drooling, salivation, spasticity, palatal myoclonus, myoclonus, myokymia, stiffness, benign myospasm, hereditary chin tremor, abnormal mandibular muscle activity, hemimasticatory spasm, hypertrophic branchial myopathy, masseter hypertrophy, tibialis anterior muscle hypertrophy, nystagmus, oscillating vision, supranuclear gaze palsy, epilepsia partialis continua, planned spasmodic torticollis surgery, abductor vocal cord paralysis, refractory mutational dysphonia, upper esophageal sphincter dysfunction, vocal cord granuloma, stuttering, Tourette syndrome, middle ear myoclonus, protective laryngeal closure, speech failure after laryngectomy, protective ptosis, entropion, sphincter of Oddi dysfunction, pseudoachalasia, non-achalasia esophageal dyskinesia, vaginismus, post-operative bed rest, tremor, bladder dysfunction, hemifacial spasm, reinnervation dyskinesia, stiff person syndrome, tetanus, prostatic hyperplasia, obesity treatment, infantile cerebral palsy, achalasia, and anal fissure.

The medical cosmetology comprises wrinkle reduction or removal, facial slimming, and scar repair.

The wrinkle may be viewed as folds, ridges, or creases in the skin. Wrinkles visible in the human face are preferred. Examples of wrinkles include lacrimal furrows, glabellar lines, crow's feet, buccal commissure lines, mandibular lines, perioral wrinkles, buccal folds, marionette lines, cheilogramma, forehead wrinkles, frown lines, bunny lines, nasolabial folds, infraocular wrinkles, and chin folds.

Compared with the prior art, the present invention has the following beneficial effects:
(1) The present invention provides a stable botulinum toxin protein composition. The composition uses amino acids or amino acid analogs to replace human protein (for example, human serum albumin), eliminating the threat of human viruses. The use of Tween 80 prevents the adsorption, providing a shelf-life of 1 year or longer in liquid state in a standard freezer (about 4 °C) or 6 months or longer in liquid state at room temperature (25 °C).
(2) The present invention provides a recombinant botulinum toxin protein composition comprising recombinant botulinum toxin type A protein at a high purity prepared from a specific single-chain polypeptide. The preparation can improve the purity of the botulinum toxin type A protein in a formulation, avoid the possible immune risk caused by the ingestion of inactive botulinum protein contained in a botulinum toxin type A substance extracted from *Clostridium botulinum* into the human body, and thus provide a new option for expanding the applications of the recombinant botulinum toxin type A protein.
(3) When used as a liquid formulation, the botulinum toxin protein composition of the present invention increases the convenience of clinical use and addresses the common shortcomings of lyophilized or vacuum-processed products, such as short shelf lives after reconstitution, susceptibility to waste and human-caused contaminations, inaccurate reconstitution, and the like.

### BRIEF DESCRIPTION OF THE DRAWINGS

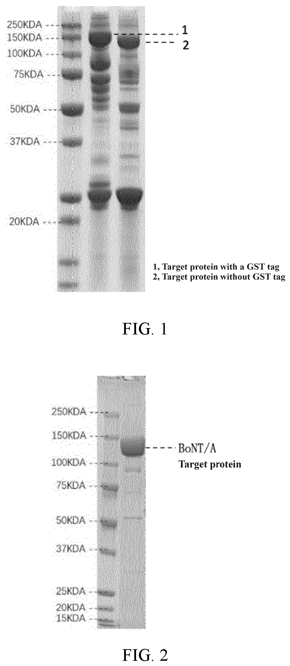

FIG. 1 illustrates the SDS-PAGE results after the preliminary purification of GST-BoNT/A and the further removal of GST tags, wherein lane 1 is GST-BoNT/A obtained in the preliminary purification by GST affinity column chromatography, and lane 2 is BoNT/A without GST obtained by removing the GST protein tag through the digestion of the preliminarily purified protein with Rinovirus 3C Protease.
FIG: 2 illustrates the SDS-PAGE results of the high-purity BoNT/A protein from further ion exchange column chromatographic purification of the product with the GST tag removed.
FIG: 3 illustrates the verification results of BoNT/A chain dissociation in a reducing condition.

### DETAILED DESCRIPTION

Unless otherwise defined, all scientific and technical terms used in the present invention have the same meaning as commonly understood by those skilled in the art to which the present invention relates.

In the present invention, the term "U" refers to a potency unit, a unit used in pharmaceutics representing the diversity of biological abilities. Since the chemical composition of some certain pharmaceuticals is not constant or their quality specifications cannot yet be determined by physicochemical methods, the potency of such pharmaceuticals is often determined by biological assays and comparison with standards. By this bioassay, the smallest potency unit with a certain biological potency is called the "unit" (U); a standard unit specified via international negotiation is called the "international unit" (IU);
The term "BoNT/A" refers to the botulinum toxin type A protein;
The term "protein tag" refers to a polypeptide or protein that is fused to and expressed with a target protein using *in vitro* DNA recombinant technology, so as to facilitate the expression and detection of the target protein.

The term "human serum albumin", abbreviated as HSA, refers to a protein in human plasma. HSA is capable of transporting fatty acids, bilirubin, amino acids, steroid hormones, metal ions, and many therapeutic molecules in body fluids, and the like. Additionally, it plays a role in maintaining the normal osmotic pressure of the blood. HSA can be used clinically to treat shock and burns, to supplement blood loss due to surgery, accidents or hemorrhage, and as a plasma expander.

The term "dimeric structure" refers to a macromolecular complex composed of two molecules, often bonded by non-covalent bonds.

The embodiments of the present invention will be clearly and completely described below with reference to the examples of the present invention. It is apparent that the described examples are only a part of the examples of the present invention, but not all of them. Based on the examples of the present invention, all other examples obtained by those of ordinary skills in the art without creative work shall fall within the protection scope of the present invention.

### Example 1 Preparation of recombinant botulinum toxin type A protein

Specifically, the process comprises the following 8 procedures:

### 1. Design and construction of nucleic acid molecule encoding the modified single-chain polypeptide of botulinum toxin:

The nucleic acid molecule comprises the following sequences sequentially from the 5' end:
(a) The nucleotide sequence encoding glutathione S-transferase is set forth in SEQ ID NO: 1, and the amino acid sequence of the encoded glutathione S-transferase is set forth in SEQ ID NO: 2;
(b) The nucleotide sequence encoding the first protease cleavage site is set forth in SEQ ID NO: 3, and the amino acid sequence of the encoded first protease cleavage site is set forth in SEQ ID NO: 4;
(c) The nucleotide sequence encoding the GS short linker peptide is set forth in SEQ ID NO: 5 and is GGATCC;
(d) The nucleotide sequence encoding the second polypeptide fragment comprising the nucleotide sequence of the BoNT/A light chain, the second protease cleavage site, and the BoNT/A heavy chain is set forth in SEQ ID NO: 6, and the encoded amino acid sequence is set forth in SEQ ID NO: 7; the nucleotide sequence encoding second protease cleavage site is set forth in SEQ ID NO: 8, and the amino acid sequence of the encoded second protease cleavage site is set forth in SEQ ID NO: 9;

There may also be no natural loop region between the light chain of BoNT/A and the heavy chain of BoNT/A. For example, the KTKSLDKGYNK linker sequence between the light chain and the heavy chain may be removed to reduce non-specific protease cleavage.

The nucleotide sequence encoding the single-chain polypeptide is set forth in SEQ ID NO: 10, and the amino acid sequence of the encoded single-chain polypeptide is set forth in SEQ ID NO: 11.

### 2. Construction of plasmid comprising the nucleic acid molecule of sequence of step 1

The genetically optimized GST-BoNT/A was artificially synthesized as described above, and NdeI and NotI enzyme cleavage sites were synthesized and added at the two ends thereof. The GST-BoNT/A was digested with the NdeI and NotI at 37 °C (New England Biolabs), purified by using QIquick gel extraction kit (Qiagen), and inserted between the NdeI and NotI sites in pET28a plasmid vector (Novagen) by using a T4 DNA ligase (NEB).

### 3. Transfection of host cells with plasmid constructed in step 2

(a) Preparation of competent cells: A tube of *E. coli* BL21 DE3 (New England Biolabs) was inoculated into a test tube containing 3 mL of LB culture medium and cultured with shaking at 37 °C overnight. On the next day, 0.5 mL of the bacterial solution was inoculated into a 250-mL flask containing 50 mL of LB culture medium and cultured with vigorous shaking (250 rpm) at 37 °C for about 3-5 h. When the OD value of the bacterial colony reached 0.3-0.4 at 600 nm, the flask was transferred to an ice bath and incubated for 10-15 min. The bacterial solution was poured into a 50-mL centrifuge tube in an aseptic condition. The solution was centrifuged at 1000 g at 4 °C for 10 min. The supernatant was discarded, and the cells were collected. 10 mL of 0.1 M CaCl₂ was added into the centrifuge tube, and the mixture was mixed well with shaking to resuspend the bacterial cells. Subsequently, the cells were incubated in an ice bath for 30 min, and centrifuged at 1000 g at 4 °C for 10 min. The supernatant was discarded, and 4 mL of 0.1 M CaCl₂ pre-cooled on ice was added to resuspend the collected bacterial cells. The cells were aliquoted at 0.2 mL/tube and preserved at 4 °C for later use within 24 h, and the remaining samples were preserved in a freezer at -70 °C.
(b) Transfection: Appropriate amounts of DNA (ng) and competent E. *coli* cells were mixed, co-incubated in an ice bath for 15 min, heat-shocked at 42 °C for 30 s, incubated in an ice bath for 5 min, shaken at 250 rpm for 1 h in a SOC medium, smeared on a plate with antibiotics, and cultured at 37 °C overnight.

### 4. Culture of host cell and induction of single-chain polypeptide expression

Culture medium composition: 11.8 g/L of tryptone, 23.6 g/L of yeast extract, 9.4 g/L of K₂HPO₄, 2.2 g/L of KH₂PO₄, and 4 mL/L of glycerol.

Culture condition: The cells were cultured with shaking at 250 rpm at 37 °C overnight. Expression induction: 1 mM IPTG was added to induce the expression at 25 °C for 5 h when the *E. coli* cells grew to a stage in which the OD600 = 1. The OD600 threshold may be selected from 0.2-1.5, the temperature threshold may be selected from 37 °C to 10 °C, and the expression time may be selected from 5-16 h.

Harvest: *E. coli* cells were collected by centrifugation at 3000 rpm for 30 min.

### 5. Identification of single-chain polypeptide expression level

A glutathione purification resin chromatographic column having a diameter of 1 cm and a height of 1 cm was prepared using Genscript glutathione purification resin, 25 mL of the lysate was slowly poured to the upper layer of the chromatographic column (be careful not to agitate the resin), and slowly directed through the column to adsorb GST-BoNT/A in the lysate for one hour. The GST-BoNT/A was eluted according to a conventional method.

The conventional method is as follows: the chromatographic column was washed with 20 column volumes of phosphate buffer, eluted with 10 column volumes of freshly prepared 10 mM glutathione eluent buffer (0.154 g reduced glutathione dissolved in 50 mL of 50 mM Tris-HCl (pH 8.0)) for eluting GST-BoNT/A, and the elution of the fusion protein was monitored by means of the absorbance reading at 280 nm. GST protein tag of the eluted protein was cleaved under the effect of Rinovirus 3C Protease. The products before and after the removal of GST protein tag were subjected to conventional SDS-PAGE. As shown in FIG. 1, compared with lane 1 without protein tag removal, the GST protein tag of lane 1 was cleaved to give a BoNT/A molecule without GST under the effect of Rinovirus 3C Protease.

Determination of proportion of GST-BoNT/A in total protein: The purified GST-BoNT/A were electrophoretically separated at 200 Volts using 4-12% SDS-PAGE (Biorad), and the predominant band at 175 kd molecular weight was GST-BoNT/A.

### 6. Removal of protein tag and purification

The product in step 5 was subjected to the removal of GST protein tag and the purification of the toxic polypeptide:
The GST-BoNT/A re-adsorbed on the glutathione purification resin chromatographic column was treated using Genscript 3C enzyme. The first enzyme cleavage site between the GST and BoNT/A was cleaved under the effect of the 3C enzyme. GST was separated. The second enzyme cleavage site between the light chain and the heavy chain of BoNT/A was then cleaved.

The glutathione purification resin chromatographic column was treated with a phosphate buffer. The light chain and the heavy chain of BoNT/A were eluted by the phosphate buffer, while the GST protein tag retained on the column was thus removed. The product after the removal of GST was subjected to conventional SDS-PAGE. As shown in FIG. 2, the obtained bands did not contain the GST protein tag, indicating that the GST protein tag had been completely removed. The SDS-PAGE results were scanned, and the purity of the obtained BoNT/A was above 90% by calculation based on the grey density of the band.

GSS, GSGS, and GGSGS polypeptides were adopted to replace the GS part of the short linker peptide, which demonstrated a similar effect to that of GS. The protein tag can be well exposed and thus completely cleaved.

### 7. Dissociation of two chains of dimeric BoNT/A in reducing condition

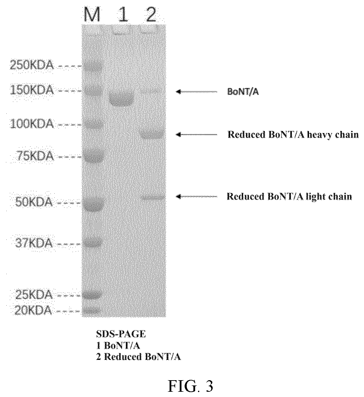

The products of step 6 were subjected to a reduction experiment:
The sample was treated by using 100 mM dithiothreitol at 100 °C for 5 min for reduction, and electrophoretically separated at 200 Volts using 4%-12% SDS-PAGE (Biorad) to separate the heavy chain and the light chain. As shown in FIG. 3, the product obtained in step 6 was reduced in the reducing condition, and the product obtained was subjected to conventional SDS-PAGE to give two different bands having molecular weights of 100 kDa and 50 kDa, respectively, suggesting that the product formed in step 6 was a dimeric structure composed of two peptide fragments linked by disulfide bonds.

### 8. Toxicity assay of GST-BoNT/A

The GST-BoNT/A obtained in step 5 exhibited an LD₅₀ of 45-450 ng after intraperitoneal injection in mice; considering the purity of the injected botulinum toxin protein, the converted LD₅₀ was 22.5-225 ng, with a median of 123.75 ng. The BoNT/A obtained in step 5 exhibited an LD₅₀ of 0.02-0.05 ng after intraperitoneal injection in mice. Considering the purity of the injected botulinum toxin protein, the converted LD₅₀ was 0.006-0.015 ng, with a median of 0.0105 ng (See Table 1).

**Table 1. Toxicity comparison of GST-BoNT/A and BoNT/A molecules in mouse biotoxicity study**

| **Test sample** | **Description** | **Dose (ng)** | **Lethality rate in mice at 72 h post-dose (%)** |
|---|---|---|---|
| GST-BoNT/A (50% purity) | GST-BoNT/A (50% purity) | 450 | 100 |
| | | 45 | 50 |
| | | 4.5 | 0 |
| | | 0.45 | 0 |
| BoNT/A (30% purity) | BoNT/A (30% purity) | 0.05 | 100 |
| | | 0.02 | 0 |
| | | 0.01 | 0 |
| | | 0.005 | 0 |

The GST-BoNT/A exhibited the activity of botulinum toxin, and the median lethal dose (LD₅₀) thereof was approximately 11786 times higher than the LD₅₀ of the BoNT/A protein after intraperitoneal injection in mice, indicating that the activity of the toxin polypeptide precursor molecule GST-BoNT/A recombinant protein is approximately 11786 times weaker than that of the final product BoNT/A. The study demonstrates that the toxin polypeptide precursor molecule of the GST-BoNT/A recombinant protein has the activity of botulinum toxin. Due to the ultra-high toxicity of botulinum toxin, high safety operation precautions are required in the manufacturing process before the activation treatment of the precursor molecule.

### Example 2 Optimization of recombinant botulinum toxin type A protein composition

The activity of the botulinum toxin protein (prepared in example 1) was assessed by the median lethal dose of mice as represented in mouse units (U). One unit is defined as the amount of the recombinant botulinum toxin type A protein required for intraperitoneal injection to kill 50% of the mice.

### I. Animal study procedures:

### 1. Species and grouping:

Adult CD-1 mice were selected, grouped, weighed, and numbered. The mice in each group were guaranteed to be equal in number and similar in body weight.

### 2. Administration:

The different prepared formulations in Table 2 were given to the mice. During the administration, 0.5 mL of recombinant botulinum toxin type A protein formulations of different compositions were given to CD-1 mice via a single intraperitoneal injection.

The amount of the recombinant botulinum toxin type A protein for intraperitoneal injection in mice was 2× LD₅₀ (2U).

### 3. Observation:

Before the start of the test, the body weight of the mice was measured once (before the administration on Day 1) and recorded. After the administration, the body weight was weighed every 24 h, and the mortality was determined 24 h (±1 h), 48 h (±1 h), 72 h (±1 h), and 96 h (±1 h) post-dose.

### II. Experimental results

The formulations (1 to 4) which contain 2U botulinum toxin listed in Table 2 were given to the mice via intraperitoneal injection. The lethality of 2U botulinum toxin in each group of mice was observed after the formulations were preserved at 4 °C for 0 days to 211 days. The procedures are the same as those of the above animal study, and the results are shown in Table 2.

**Table 2. Activity assay of formulations after different periods of preservation**

| No. | Composition | Period of preservation at 4 °C (days) | | | | | |
|---|---|---|---|---|---|---|---|
| | | 0 | 9 | 36 | 44 | 75 | 211 |
| 1 | 2U recombinant botulinum toxin type A, 0.1% Tween 80, 10 mM L-carnitine, and normal saline | 80% | 80% | 80% | 80% | 70% | 90% |
| 2 | 2U recombinant botulinum toxin type A, 0.1% Tween 80, 10 mM L-tryptophan, and normal saline | 80% | 70% | 80% | 90% | 80% | - |
| 3 | 2U recombinant botulinum toxin type A, 0.1% Tween 80, 10 mM L-histidine, and normal saline | 80% | 100% | 80% | - | - | - |
| 4 | 2U recombinant botulinum toxin type A, 0.1% Tween 80, 10 mM L-methionine, and normal saline | 80% | 80% | 70% | - | - | - |

As can be seen from the results in Table 2, the lethal effect (pharmaceutical activity) of the recombinant botulinum toxin type A in mice was not weakened after the 4 formulations in the table were preserved at 4 °C for 0 days to 211 days, and the biological activity of the recombinant botulinum toxin type A protein before the preservation at 4 °C was retained, demonstrating that the selected formulations of the present invention can maintain the long-term stability of the recombinant botulinum toxin type A.

Two formulations from Table 2 were selected to determine the median lethal dose (LD₅₀) in mice after the recombinant botulinum toxin type A was preserved at 4 °C for 44 days, 75 days, and 211 days. The results are shown in Table 3.

**Table 3. Determined LD₅₀ in mice of recombinant botulinum toxin type A after preservation at 4 °C for 44 days, 75 days, and 211 days**

| No. | Formulation | Day 44 | Day 70 | Day 211 |
|---|---|---|---|---|
| 1 | 2U recombinant botulinum toxin type A, 0.1% Tween 80, 10 mM L-carnitine, and normal saline | 11.0 pg | 11.4 pg | 12.5 pg |
| 2 | 2U recombinant botulinum toxin type A, 0.1% Tween 80, 10 mM L-histidine, and normal saline | 13.7 pg | 13.5 pg | - |

As can be seen from Table 3, the activity of the recombinant botulinum toxin type A protein was retained after formulation 1 was preserved at 4 °C for 211 days, while the activity of the recombinant botulinum toxin type A protein was retained after formulation 2 was preserved at 4 °C for 75 days. The calculated median lethal dose (LD₅₀) in mice of the recombinant botulinum toxin type A by the multi-dose-activity relation has no significant change after preservations at 4 °C for 44 days, 75 days and 211 days, and the median lethal dose was basically unchanged, demonstrating that the formulations of the present invention can effectively maintain the biological activity of the recombinant botulinum toxin type A for a long time.

According to the present invention, the recombinant botulinum toxin can be preserved for a long time (for example, 75 days or longer at 4 °C) at a low temperature (for example, at 4 °C) using amino acids or amino acid analogs as the protein stabilizer. The formulation of the present invention can also retain the activity of natural botulinum toxin proteins (botulinum toxin type A protein, botulinum toxin type B protein, botulinum toxin type C1 protein, botulinum toxin type C2 protein, botulinum toxin type D protein, and botulinum toxin type E protein).

The above description is only intended to illustrate the preferred examples of the present invention, rather than limit the scope of the present invention. Any modifications, equivalent replacements, and the like that are made within the spirit and principle of the present invention shall all fall within the protection scope of the present invention.

The foregoing examples and methods described herein can vary based on the abilities, experience, and preferences of those skilled in the art.

The certain order in which the steps of the method are listed in the present invention does not constitute any limitation to the order of the steps of the method.

## Claims

1. A botulinum toxin protein composition, comprising:
a botulinum toxin protein and a protein stabilizer,
wherein the protein stabilizer is selected from any one of an amino acid or an amino acid analog.

2. The composition according to claim 1, wherein the amino acid analog includes L-carnitine.

3. The composition according to claim 2, wherein the molar content of L-carnitine is 5-20 mM.

4. The composition according to claim 1, wherein the molar content of the amino acid is 5-20 mM.

5. The composition according to claim 4, wherein the amino acid is selected from one or more of L-histidine, L-glycine, L-arginine, L-methionine,L-tryptophan and L-lysine.

6. The composition according to any one of claims 1-5, wherein the botulinum toxin protein is selected from any one of botulinum toxin type A protein, botulinum toxin type B protein, botulinum toxin type C1 protein, botulinum toxin type C2 protein, botulinum toxin type D protein, botulinum toxin type E protein, botulinum toxin type F protein, botulinum toxin type G protein, or botulinum toxin type H protein; preferably, the botulinum toxin protein is botulinum toxin type A protein, more preferably, the botulinum toxin is a recombinant botulinum toxin.

7. The composition according to any one of claims 1-6, further comprising a solubilizer and/or a salt.

8. The composition according to claim 7, wherein the volume percentage concentration of the solubilizer is 0.01%-0.2%.

9. The composition according to claim 7 or 8, wherein the mass percentage of the salt is 0.9%.

10. The composition according to any one of claims 7-9, wherein the solubilizer is selected from a Tween, a Span, and sodium dodecyl sulfate;
preferably, the Tween is selected from one or more of Tween 20, Tween 40, Tween 60, or Tween 80;
preferably, the Span is selected from one or more of Span 20, Span 40, Span 60, Span 80, or Span 85;
more preferably, the solubilizer is Tween 80.

11. The composition according to any one of claims 7-10, wherein the salt is selected from any one of an inorganic salt or an organic salt;
preferably, the inorganic salt is selected from one or more of sodium chloride, sodium phosphate, magnesium sulfate, sodium acetate, sodium propionate, and tripotassium phosphate;
preferably, the organic salt is selected from one or more of sodium lactate and sodium succinate;
more preferably, the inorganic salt is sodium chloride.

12. The composition according to any one of claims 1-11, wherein the composition comprises the botulinum toxin protein, L-carnitine, Tween 80, and sodium chloride; or,
the composition comprises the botulinum toxin protein, the amino acid, Tween 80, and sodium chloride.

13. The composition according to claim 12, wherein the volume percentage concentration of Tween 80 is 0.05%-0.2%;
preferably, the volume percentage concentration of Tween 80 is 0.1%.

14. The composition according to claim 12 or 13, wherein the mass percentage concentration of sodium chloride is 0.9%.

15. The composition according to any one of claims 1-14, wherein the composition is a liquid formulation.

16. A method for preparing the composition according to any one of claims 1-15, comprises a step of mixing the components.

17. Use of the composition according to any one of claims 1-15 in preparing a medicament for preventing and/or treating a disease associated with cholinergic nerves innervating muscles or exocrine glands,
or,
in preparing a medicament for medical cosmetology;
wherein preferably, the medical cosmetology comprises wrinkle reduction or removal, facial slimming, body contouring, and scar repair; preferably, the disease associated with cholinergic nerves is selected from:
one or more of Frey's syndrome, crocodile tears syndrome, armpit hyperhidrosis, plantar hyperhidrosis, head and neck hyperhidrosis, body hyperhidrosis, rhinorrhea, Parkinson's disease, amyotrophic lateral sclerosis, sialorrhea, drooling, salivation, spasticity, palatal myoclonus, myoclonus, myokymia, stiffness, benign myospasm, hereditary chin tremor, abnormal mandibular muscle activity, hemimasticatory spasm, hypertrophic branchial myopathy, masseter hypertrophy, tibialis anterior muscle hypertrophy, nystagmus, oscillating vision, supranuclear gaze palsy, epilepsia partialis continua, planned spasmodic torticollis surgery, abductor vocal cord paralysis, refractory mutational dysphonia, upper esophageal sphincter dysfunction, vocal cord granuloma, stuttering, Tourette syndrome, middle ear myoclonus, protective laryngeal closure, speech failure after laryngectomy, protective ptosis, entropion, sphincter of Oddi dysfunction, pseudoachalasia, non-achalasia esophageal dyskinesia, vaginismus, post-operative bed rest, tremor, bladder dysfunction, hemifacial spasm, reinnervation dyskinesia, stiff person syndrome, tetanus, prostatic hyperplasia, obesity treatment, infantile cerebral palsy, achalasia, and anal fissure.
